# EUROPEAN PATENT SPECIFICATION

(11) **EP 0 934 692 B1**
(45) Date of publication and mention of the grant of the patent: **23.10.2002**
(21) Application number: 99200403.6
(22) Date of filing: 31.01.1995
(51) Int. Cl.: A01H 5/10, A01H 1/06

(54) **Sunflower seeds and plants having a high stearic acid content**
Sonnenblumensamen und Pflanzen mit hohem Stearinsäuregehalt
Graines et plantes de tournesol à teneur élevée en acide stéarique

(30) Priority: 31.01.1994 ES 9400177; 31.01.1994 ES 9400178; 24.06.1994 ES 9401383; 24.06.1994 ES 9401384
(43) Date of publication of application: 11.08.1999
(62) Divisional of application: 95908894.9
(73) Proprietor: CONSEJO SUPERIOR DE INVESTIGACIONES CIENTIFICAS, E-41002 Sevilla (ES)
(72) Inventor: Garcés, Rafael, 41907 Valencina de la Concepcion (ES); Mancha, Manuel, 41013 Sevilla (ES); Osorio, Jorge, 41011 Sevilla (ES); Fernandez, José Maria, 14012 Cordoba (ES)
(74) Representative: Van Someren, Petronella F. H. M.

(56) References cited:
- EP-A- 0 431 833
- EP-A- 0 496 504
- US-A- 4 378 655
- US-A- 4 627 192
- PATENT ABSTRACTS OF JAPAN vol. 014, no. 197, 23 April 1990 & JP 02 039834 A (KUMAO KATSUKI), 8 February 1990

## Description

The present invention relates to sunflower seeds comprising an oil having an increased stearic acid content as compared to wild type plants between 10% and 35% by weight related to the total amount of fatty acids in the oil. The invention also relates to sunflower seeds having a stearic acid content up to 54% by weight or more. The invention further relates to sunflower plants produced from the seeds.

Sunflower is generally cultivated for obtaining oil which has saturated fatty acids (palmitic and stearic) and unsaturated fatty acids (oleic and linoleic). The stearic acid content is always less than 10% (Gustone, F.D. et al. "The lipid handbook"; Chapman and Hall 1986), normally comprised between 3% and 7%. In relation with the unsaturated fatty acids there are two different kinds of sunflower seeds: the normal sunflower which has a linoleic acid content between 50% and 70% (Knowles, P.F. "Recent advances in oil crops breeding"; AOCS Proceedings 1988) and the high oleic sunflower which has 2-10% of linoleic acid and 75-90% of oleic acid (Soldatov, K.I. "Chemical mutagenesis in sunflower breeding"; Int. Proc. 7th Intern. Sunflower Conference, 352-357, 1976). There is also a sunflower line having a high palmitic acid content, between 22% and 40% (R. Ivanov et al. "Sunflower Breeding for High Palmitic Acid Content in the Oil; Proc. of the 12th Intern. Sunflower Conference, Vol. II, 453-465, 1988) and another line with low saturated fatty acid content (6% or less) (EP-A-496504).

Table 1 shows the fatty acid composition for some known sunflower oil varieties.

**Table 1**

| **% of fatty acids in sunflower oil** | | | | |
|---|---|---|---|---|
| Variety | Palmitic | Stearic | Oleic | Linoleic |
| Normal¹ | 5.9 | 5.7 | 21.8 | 66.5 |
| High oleic¹ | 3.1 | 4.8 | 84.9 | 6.7 |
| Low saturated² | 3.9 | 2.2 | 89.9 | 4.0 |
| High palmitic³ | 25.1 | 4.3 | 10.6 | 56.4 |

| | | | | |
|---|---|---|---|---|
| ¹ Fernández Martínez et al.; Grasas y Aceites 37, (1986) | | | | |
| ² Patent EP-A-496504 | | | | |
| ³ This variety has also 3.6% of palmitoleic acid | | | | |

From US-A-4 627 192 are known sunflower seeds with a stearic acid content of 10% in the oil.

The saturated fatty acid content of an oil is directly related with the physical and chemical characteristics thereof. In case that said content is sufficiently high, the oil can be a solid at room temperature like some animal fats. Normal sunflower oil is always a liquid under said conditions.

In the food industry like for the production of confectionery or margarine, animal fats or hydrogenated vegetable fats are usually used because a solid or semisolid product is required. By means of hydrogenation unsaturated fatty acids are converted into saturated fatty acids. Animal fats as well as hydrogenated fats are not very recommendable from a nutritional point of view (Chow, C.K. "Fatty acids in food and their health implications", Dekker, N.Y., 1992). Animal fats have a relatively high cholesterol content. Too much cholesterol in the diet may be detrimental to the health. Therefore animal fats have been substituted in the last years by hydrogenated vegetable fats which do not contain cholesterol.

However, said hydrogenated fats present another problem derived from the hydrogenation process. In said process positional isomerization (shift of double bonds) and stereo-chemical transformations (formation of "trans" isomers) take place. Isomers are produced in an amount of up to 30%-50% of the total fatty acids amount. These isomers are not very healthy from a nutritional point of view (Wood, R., "Biological effects of geometrical and positional isomers of monounsaturated fatty acids in humans"; Dekker, N.Y. (1990); Willet, W.C. & Ascherio, A., "Trans Fatty Acids: Are The Effects Only Marginal ?", American Journal of Public Health, Vol. 84, 5, (1994)). Therefore, the use of hydrogenated fats in the food industry should be avoided.

Sunflower oil has a desirable content of unsaturated fatty acids. For use in the food industry however, the stearic acid content of the oil must be higher than in the normal sunflower oil (Norris, M.E., "Oil substitutions in food formulations", Inform. 1, 388-392 (1990)) in order to obtain a more solid product.

It is thus an object of the invention to provide new mutated seeds, the oil of which having a higher stearic acid content as compared to oil obtained from wild type seeds.

The invention therefore provides sunflower seeds, comprising a sunflower oil having a stearic acid content of at least 12% by weight related to the total amount of fatty acid in the oil, which seeds are obtainable by treating parent seeds with a mutagenic agent during a period of time and in a concentration sufficient to induce one or more mutations in the genetic trait involved in stearic acid biosynthesis resulting in an increased production of stearic acid as compared to the parents, germinating the treated seeds and culturing progeny plants therefrom, collecting and analyzing progeny seeds, selecting seeds that have acquired the desirable genetic trait and optionally repeating the cycle of germination, culturing and collection of seeds.

Preferably the sunflower seeds according to the invention comprise an oil having a stearic acid content of between 19.1 and 35% by weight, related to the total amount of fatty acids in the oil, and are obtainable by treating the parent seeds during 2 hours at room temperature with an alkylating agent such as a solution of 70 mM ethyl methane sulfonate in water.

In another embodiment of the invention the seeds comprise an oil having a stearic acid content of between 10 and 19% by weight related to the total amount of fatty acids in the oil, and are obtainable by treating the parent seeds with a solution of 2 mM sodium azide in water during 2 hours at room temperature.

Sunflower seeds identified as "CAS-3" having an average stearic acid content of 25% by weight, related to the total amount of fatty acids in the oil, have been deposited on December 14, 1994 with the American Type Culture Collection, 12301 Parklawn Drive, Rockville, MD 20852, U.S.A. under deposit accession number ATCC 75968. Sunflower seeds identified as "CAS-4" having an average stearic acid content of 15.4% by weight, related to the total amount of fatty acids in the oil, have been deposited on the same day with the same institution under deposit accession number ATCC 75969.

Seeds having an even higher stearic acid content between 29 and 54% by weight related to the total amount of fatty acids in the oil, may be obtained according to the invention by crossing sunflowers originating from seeds having a stearic acid content between 19.1 and 35% by weight with sunflowers originating from seeds having a stearic acid content between 10 and 19% by weight, and collecting the seeds.

Preferably the sunflower oil of the seeds of the invention has a palmitic acid content between 3 and 40% by weight, an oleic acid content between 3 and 85% by weight and a linoleic acid content between 2 and 84% by weight, all related to the total amount of fatty acids in the oil. Such types of oil may be obtained from seeds produced by crossing the high stearic acid seeds of the invention with seeds having a desirable content of one or more unsaturated and/or saturated fatty acids. Thus tailor-made seeds and tailor-made oil produced therefrom may be obtained by preparing mutants according to the invention and use these in further conventional plant improvement practice by crossing them with other known or as yet unknown mutant or wild type plants.

The seeds of the invention are prepared by a method for preparing sunflower seeds having an increased stearic acid content as compared to wild type seeds, by treating parent seeds with a mutagenic agent during a period of time and in a concentration sufficient to induce one or more mutations in the genetic trait involved in stearic acid biosynthesis resulting in an increased production of stearic acid, germinating the treated seeds and culturing progeny plants therefrom, collecting and analyzing progeny seeds, selecting seeds that have acquired the desirable genetic trait and optionally repeating the cycle of germination, culturing and collection of seeds.

In practice the method comprises mutagenesis of sunflower seeds with a suitable mutagenic agent. The mutagenesis will produce inheritable genetic changes in the DNA of the seeds. According to the invention it was possible after several different treatments to select some treatments that produced a high number of genetic modifications in the genes that control the seed fatty acid biosynthesis. These treatments comprise the use of sodium azide or an alkylating agent, like ethyl methane sulfonate. Of course any other mutagenic agent having the same or similar effects may also be used.

Then, the next seed generation was analyzed with a new methodology described in Garcés, R. and Mancha, M. "One-step lipid extraction and fatty acid methyl esters preparation from fresh plant tissues". Analytical Biochemistry, 211:139-143, 1993. This allowed for the detection of seeds with modifications in the composition of any fatty acid. Selected seeds showing a desirably high stearic acid content have been cultivated to the fifth generation showing that this new genetic trait is inheritable and stable and independent of growth conditions.

In the method the parent seeds are for example treated during 2 hours at room temperature with a solution of 70 mM ethyl methane sulfonate in water, or during 2 hours at room temperature with a solution of 2 mM sodium azide in water.

In a further embodiment of the method, the mutation and selection steps may be followed by conventional plant improvement techniques thus leading to seeds having e.g. an even higher stearic acid content up to 54% by weight or more, or to seeds having a desirable content of one or more other fatty acids. In still another embodiment the seeds of the invention may be subjected to one or more further mutation treatments.

Sunflower oil having a stearic acid content of between 10 and 35% by weight, related to the total amount of fatty acids in the oil, may be prepared by extraction from sunflower seeds of the invention in any manner known to the person skilled in the art. Such extraction methods are well known and for example described in "Bailey's industrial oil and fat products", Vol. 2, Chapter 3; 4th Edition, John Wiley and Sons, New York (1982).

The invention further relates to sunflower plants produced from seeds according to the invention. Thus, the seeds can be used to produce parent lines that have high stearic acid content in their oil. This also applies to plants originating from seeds obtained after crossing the mutants of the invention with each other or with other seeds having a desirable phenotype. The seeds may be cultured in the normal way on soil or any other substrate. The production of the modified plants does not require any additional measure as compared to the growing of ordinary sunflower seeds.

The sunflower plants may be used in breeding programmes for the development of sunflower lines or hybrids, which programmes are aimed at the production of open pollinated or hybrid varieties meeting the requirements of farming practice regarding yield, disease resistance and other agronomically important traits in major sunflower growing areas in the world. Seeds resulting from these programmes may be used in the growing of commercial sunflower crops.

The sunflower oil of the seeds of the invention may be used in the food industry. The natural vegetable oil that has been extracted from mutagenized sunflowers seeds has a high stearic acid content between 10 and 35%, or in the case of intercrossing of the seeds, even up to 54% or more. This allows to use the oil from these kinds of seeds as such. However, combinations of the oil of the seeds of the invention with oil from the known high oleic acid or high palmitic acid sunflower seeds, in the production of edible fats or fat mixtures, like margarine, vegetable-dairy or in the production of confectionery or bakery is also possible depending on the requirements of the application. The advantage of these oils is that they do not have artificial fatty acid isomers, like the "trans" isomers found in the hydrogenated oils, and, of course, no cholesterol, like in the animal fats.

The oil of the seeds of the invention may be used in products, such as margarine, vegetable-dairy, confectionery or bakery. The oil may simply replace oils or fats ordinarily used in this type of products. It is within the reach of the skilled person to determine how to use the oil without performing any inventive labor.

The present invention will be further illustrated by means of the following examples which are given for illustration purposes only and are in no way intended to limit the scope of the invention.

### EXAMPLES

### Materials and methods

Sodium azide and ethyl methane sulfonate were used as mutagenic agents in Example 1 and 2, respectively. Several sunflower lines with a stearic acid content between 10 and 35% have been obtained. In all these cases the original sunflower parent line used was RDF-1-532 (Sunflower Collection of Instituto de Agricultura Sostenible, CSIC, C6rdoba, Spain) that has from 4 to 7% stearic acid content in the seed oil. The preparation of the lines CAS-3 and CAS-4, and of the line CAS-3x4 obtained after crossing CAS-3 with CAS-4, have been described in the following examples.

### EXAMPLE 1

Seeds were mutagenized with a solution of 70 mM of ethyl methane sulfonate (EMS) in water. The treatment was performed at room temperature during 2 hours while shaking (60 rpm). After mutagenesis the EMS solution was discarded and seeds were washed during 16 hours under tap water.

Treated seeds were germinated in the field and plants were self-pollinated. The seeds collected from these plants were used to select new sunflower lines with modifications in the fatty acid composition. By using the method of Garcés, R. and Mancha, M. (supra) the seed fatty acid composition was determined by gas liquid chromatography, after converting the fatty acids into their corresponding methyl esters.

A first plant with 9 to 17% stearic acid content in the oil was selected. The progeny was cultivated for five generations wherein the stearic acid content increased and the new genetic trait became stably fixed in the genetic material of the seed. This line is called CAS-3. A selected sample of this line was analyzed resulting in a stearic acid content of 26% (Table 2). The minimum and the maximum stearic acid content of the line were 19 and 35% respectively. The stearic acid content of oil extracted from seeds from this cell line may thus lie between 19 and 35%.

### EXAMPLE 2

Sunflower seeds were mutagenized with sodium azide, at a concentration of 2 mM in water. The treatment was performed at room temperature during two hours while shaking (60 rpm). Then the mutagenesis solution was discarded and seeds were washed during 16 hours with tap water.

Seeds were planted in the field and plants were self-pollinated. Seeds from these plants were collected, and the fatty acid composition was determined by gas liquid chromatography, after converting the fatty acids into their corresponding methyl esters using the method described in Example 1.

Seeds from a plant having around 10% stearic acid in the oil were selected and cultivated for five generations. During this procedure the stearic acid content was increased and the new genetic trait fixed. This line is called CAS-4. A selected sample of this line was analyzed resulting in a stearic acid content of 16.1%. The minimum and the maximum values were 12 and 19% respectively (Table 2).

**Table 2**

| **Percentage fatty acids** | | | | |
|---|---|---|---|---|
| Line | Palmitic | Stearic | Oleic | Linoleic |
| RDF-1-532 | 6.7 | 4.5 | 37.4 | 51.3 |
| CAS-3 | 5.1 | 26.0 | 13.8 | 55.1 |
| CAS-4 | 5.5 | 16.1 | 24.3 | 54.1 |

### EXAMPLE 3

Sunflower plants were grown from the sunflower seeds CAS-3 and CAS-4. The plants thus obtained were artificially pollinated in order to ensure only crossings between CAS-3 and CAS-4 to occur, not pollination of the mutant plants amongst themselves.

From the seeds thus produced plants were grown and the stearic acid content of the progeny was determined as described in Examples 1 and 2. The hybrid CAS-3x4 had a stearic acid content of more than 35% by weight. From this it appears that intercrossing the mutants will yield hybrids with an even higher stearic acid content.

According to the invention sunflower plants and seeds from which said oil can be extracted have been obtained by means of a biotechnological process. This high stearic acid content is an inheritable trait and is independent from the growing conditions.

## Claims

1. Sunflower seeds, comprising a sunflower oil having a stearic acid content of at least 12% by weight related to the total amount of fatty acid in the oil, which seeds are obtainable by treating parent seeds with a mutagenic agent during a period of time and in a concentration sufficient to induce one or more mutations in the genetic trait involved in stearic acid biosynthesis resulting in an increased production of stearic acid as compared to the parent seeds, germinating the treated seeds and culturing progeny plants therefrom, collecting and analyzing progeny seeds, selecting seeds that have acquired the desirable genetic trait and repeating the cycle of germination, culturing and collection of seeds.

2. Sunflower seeds as claimed in claim 1, **characterized in that** the seeds comprise an oil having a stearic acid content of between 19.1 and 35% by weight related to the total amount of fatty acids in the oil, and are obtainable by treating the parent seeds with an alkylating agent.

3. Sunflower seeds as claimed in claim 2, **characterized in that** the parent seeds are treated during 2 hours at room temperature with a solution of 70 mM ethyl methane sulfonate in water.

4. Sunflower seed as claimed in claim 1, **characterized in that** the seeds comprise an oil having a stearic acid content of between 12 and 19% by weight related to the total amount of fatty acids in the oil, and are obtainable by treating the parent seeds with a solution of 2 mM sodium azide in water.

5. Sunflower seed as claimed in claims 1, 2 and 3, **characterized in that** the seeds comprise an oil having a stearic acid content of 25% by weight related to the total amount of fatty acids in the oil.

6. Sunflower seed as claimed in claim 5, obtainable from the American Type Culture Collection under deposit accession number ATCC 75968.

7. Sunflower seed as claimed in claim 1 and 4, **characterized in that** the seeds comprise an oil having a stearic acid content of 15.4% by weight related to the total amount of fatty acids in the oil.

8. Sunflower seed as claimed in claim 7, obtainable from the American Type Culture Collection under deposit accession number ATCC 75969.

9. Sunflower seed according to claim 1 having a stearic acid content between 29 and 54% by weight related to the total amount of fatty acids in the oil, obtainable by crossing sunflowers originating from seeds according to claims 2, 3, 5 and 6 with sunflowers originating from seeds according to claims 4, 7 and 8 and collecting the seeds.

10. Sunflower seed having a stearic acid content as claimed in any one of the claims 1-9, and in addition a palmitic acid content between 3 and 40% by weight or an oleic acid content between 3 and 85% by weight or a linoleic acid content between 2 and 84% by weight, all related to the total amount of fatty acids in the oil, or any combination of one or more of these fatty acid contents, obtainable by crossing plants originating from the mutant seeds according to claims 1-9 with a plant showing a desired phenotype with respect to its fatty acid content.

11. Sunflower plant produced from seeds as claimed in any one of the claims 1-10.

## Patentansprüche

1. Sonnenblumensamen, die ein Sonnenblumenöl mit einem Stearinsäuregehalt von mindestens 12 Gew.-% bezogen auf die Gesamtmenge an Fettsäure im Öl umfassen, wobei die Samen erhältlich sind durch Behandeln von Muttersamen mit einem Mutagen während einer Zeitdauer und in einer Konzentration, die ausreichend ist zur Induktion einer oder mehrerer Mutationen in einem genetischen Merkmal, welches in der Stearinsäurebiosynthese involviert ist, was zu einer erhöhten Produktion an Stearinsäure im Vergleich zu den Muttersamen führt, Keimen der behandelten Samen und Kultivieren der nachkommenden Pflanzen daraus, Einsammeln und Analysieren der nachkommenden Samen, Einsammeln der Samen, die das gewünschte genetische Merkmal erworben haben, und Wiederholen des Zyklus der Keimung, Kultivierung und Einsammlung der Samen.

2. Sonnenblumensamen wie im Anspruch 1 beansprucht, **dadurch gekennzeichnet, dass** die Samen ein Öl mit einem Stearinsäuregehalt zwischen 19,1 und 35 Gew.-% bezogen auf die Gesamtmenge an Fettsäuren im Öl umfassen und erhältlich sind durch Behandeln der Muttersamen mit einem Alkylierungsmittel.

3. Sonnenblumensamen wie im Anspruch 2 beansprucht, **dadurch gekennzeichnet, dass** die Muttersamen während zwei Stunden bei Raumtemperatur mit einer Lösung von 70 mM Äthylmethansulfonat in Wasser behandelt wurden.

4. Sonnenblumensamen wie im Anspruch 1 beansprucht, **dadurch gekennzeichnet, dass** die Samen ein Öl mit einem Stearinsäuregehalt zwischen 12 und 19 Gew.-% bezogen auf die Gesamtmenge an Fettsäuren im Öl umfassen und erhältlich sind durch Behandeln der Muttersamen mit einer Lösung von 2 mM Natriumazid in Wasser.

5. Sonnenblumensamen wie in den Ansprüchen 1, 2 und 3 beansprucht, **dadurch gekennzeichnet, dass** die Samen ein Öl mit einem Stearinsäuregehalt von 25 Gew.-% bezogen auf die Gesamtmenge an Fettsäuren im Öl umfassen.

6. Sonnenblumensamen wie im Anspruch 5 beansprucht, erhältlich von der American Type Culture Collection unter der Hinterlegungseingangsnummer ATCC 75968.

7. Sonnenblumensamen wie im Anspruch 1 und 4 beansprucht, **dadurch gekennzeichnet, dass** die Samen ein Öl mit einem Stearinsäuregehalt von 15,4 Gew.-% bezogen auf die Gesamtmenge an Fettsäuren im Öl umfassen.

8. Sonnenblumensamen wie im Anspruch 7 beansprucht, erhältlich von der American Type Culture Collection unter der Hinterlegungseingangsnummer ATCC 75969.

9. Sonnenblumensamen gemäß Anspruch 1 mit einem Stearinsäuregehalt zwischen 29 und 54 Gew.-% bezogen auf die Gesamtmenge an Fettsäuren im Öl, erhältlich durch Kreuzen von Sonnenblumen, die aus Samen gemäß den Ansprüchen 2, 3, 5 und 6 stammen, mit Sonnenblumen, die von Samen gemäß den Ansprüchen 4, 7 und 8 stammen, und Einsammeln der Samen.

10. Sonnenblumensamen mit einem Stearinsäuregehalt wie in irgendeinem der Ansprüche 1 bis 9 beansprucht sowie zusätzlich einem Palmitinsäuregehalt zwischen 3 und 40 Gew.-% oder einem Ölsäuregehalt zwischen 3 und 85 Gew.-% oder einem Linolsäuregehalt zwischen 2 und 84 Gew.-%, jeweils bezogen auf die Gesamtmenge an Fettsäuren im Öl, oder irgendeiner Kombination von einem oder mehreren dieser Fettsäuregemengen, erhältlich durch Kreuzen von Pflanzen, die aus Mutationssamen gemäß den Ansprüchen 1 bis 9 stammen, mit einer Pflanze, die einen gewünschten Phänotyp in Bezug auf ihren Fettsäuregehalt zeigt.

11. Sonnenblumenpflanze, die aus Samen wie in irgendeinem der Ansprüche 1 bis 10 beansprucht produziert wurde.

## Revendications

1. Semences de tournesol, comprenant une huile de tournesol ayant une teneur en acide stéarique d'au moins 12 % en poids par rapport à la quantité totale de l'acide gras daps l'huile, lesquelles semences peuvent être obtenues par traitement de semences parentes avec un agent mutagénique pendant un laps de temps et à une concentration suffisants pour induire une ou plusieurs mutations dans le caractère génétique mis en jeu dans la biosynthèse de l'acide stéarique, avec pour conséquence une production accrue d'acide stéarique pax comparaison aux semences parentes, germination des semences traitées et culture des plantes qui en sont la descendance, collecte et analyse des semences descendantes, sélection des semences ayant acquis le caractère génétique recherché, et répétition du cycle de germination, culture et collecte des semences.

2. Semences de tournesol selon la revendication 1, **caractérisées en ce que** les semences comprennent une huile ayant une teneur en acide stéarique comprise entre 19,1 et 35 % en poids par rapport à la quantité totale des acides gras danns l'huile, et peuvent être obtenues par traitement des semences parentes avec un agent d'alkylation.

3. Semences de tournesol selon la revendication 2, **caractérisées en ce que** les semences parentes sont traitées pendant 2 heures à la température ambiante avec une solution de 70 mM de méthanesulfonate d'éthyle dans l'eau.

4. Semences de tournesol selon la revendication 1, **caractérisées en ce que** les semences comprennent une huile ayant une teneur en acide stéarique comprise entre 12 et 19 % en poids par rapport à la quantité totale des acides gras danns l'huile, et peuvent être obtenues par traitement des semences parentes avec une solution de 2 mM d'azoture de sodium dans l'eau.

5. Semences de tournesol selon les revendications 1, 2 et 3, **caractérisées en ce que** les semences comprennent une huile ayant une teneur en acide stéarique de 25 % en poids par rapport à la quantité totale des acides gras dans l'huile.

6. Semences de tournesol selon la revendication 5, pouvant être obtenues auprès de l'America Type Culture Collection sous le numéro d'enregistrement de dépôt ATCC 75968.

7. Semences de tournesol selon les revendications 1 et 4, **caractérisées en ce que** les semences comprennent une huile ayant une teneur en acide stéarique de 15,4 % en poids par rapport à la quantité totale des acides gras dans l'huile.

8. Semences de tournesol selon la revendication 7, pouvant être obtenues auprès de l'American Type Culture Collection sous le numéro d'enregistrement de dépôt ATCC 75969.

9. Semences de tournesol selon la revendication 1, ayant une teneur en acide stéarique comprise entre 29 et 54 % en poids par rapport à la quantité totale des acides gras dans l'huile, pouvant être obtenues par croisement de tournesols provenant de semences selon les revendication 2, 3, 5 et 6 avec des tournesols provenant des semences selon les revendications 4, 7 et 8, et collecte des semences.

10. Semences de tournesol ayant une teneur en acide stéarique selon l'une quelconque revendication 1 à 9, et en outre une teneur en acide palmitique comprise entre 3 et 40 % en poids ou une teneur en acide oléique comprise entre 3 et 85 % en poids ou une teneur en acide linoléique comprise entre 2 et 84 % en poids, toutes étant rapportées à la quantité totale des acides gras dans l'huile, ou une combinaison quelconque d'une ou plusieurs de ces teneurs en acide gras, pouvant être obtenues par croisement de plantes provenant des semences mutantes selon les revendications 1 à 9 avec une plante présentant un phénotype voulu pour ce qui concerne sa teneur en acide gras.

11. Plant de tournesol produit à partir de semences selon l'une quelconque des revendications 1 à 10.
